(19) Europäisches Patentamt

European Patent Office

Office européen des brevets

(11) Veröffentlichungsnummer: **0 226 172 B1**

(12) **EUROPÄISCHE PATENTSCHRIFT**

(51) Int. Cl.⁵: **A61K 37/64**, //(A61K37/64, 37:547),(A61K37/64,37:54), (A61K37/64,37:465)

(45) Veröffentlichungstag der Patentschrift: **20.05.92**

(21) Anmeldenummer: **86117169.2**

(22) Anmeldetag: **09.12.86**

(54) **Mittel aus einem Plasmin-Inhibitor und einem Plasminogen-Aktivator zur Behandlung von Thrombosen.**

(30) Priorität: **14.12.85 DE 3544318**

(43) Veröffentlichungstag der Anmeldung:
**24.06.87 Patentblatt 87/26**

(45) Bekanntmachung des Hinweises auf die Patenterteilung:
**20.05.92 Patentblatt 92/21**

(84) Benannte Vertragsstaaten:
**BE CH DE ES FR GB IT LI NL SE**

(56) Entgegenhaltungen:

BIOLOGICAL ABSTRACTS, Band 64, 1. Dezember 1977, Seite 5963, Ref.Nr. 60894, Philadelphia, US; L. SUMMARIA et al.: "Activation of human Glu-plasminogen to Glu-plasmin by urokinase in presence of plasmin inhibitors: Streptomyces leupeptin and human plasma alpha-1-antitrypsin and antithrombin III (plus heparin)", & J. BIOL. CHEM., 252(11), 3945-3951, 1977

Idem

(73) Patentinhaber: **BEHRINGWERKE Aktiengesellschaft**
**Postfach 1140**
**W-3550 Marburg 1(DE)**

(72) Erfinder: **Paques, Eric Paul, Dr.**
**Schmiedeacker 18**
**W-3550 Marburg(DE)**
Erfinder: **Karges, Hermann, Dr.**
**Sonnenweg 32**
**W-3550 Marburg(DE)**

(74) Vertreter: **Klein, Otto, Dr. et al**
**Hoechst AG Zentrale Patentabteilung Postfach 80 03 20**
**W-6230 Frankturt am Main 80(DE)**

CHEMICAL ABSTRACTS, Band 102, Nr. 7, 18. Februar 1985, Seite 405, Ref.Nr. 59969a, Columbus, Ohio, US; T.T. CHIEN et al.: "Tissue-type plasminogen activator increases the binding of Glu-plasminogen to clots", & J. CLIN. INVEST. 1984, 74(6), 2009-16

BIOLOGICAL ABSTRACTS, Band 78, Nr. 2, 1984, Seite 1548, Ref.Nr. 13532, Philadelphia, US; F. VAN DE WERF et al.: "Coronary thrombolysis with tissue-type plasminogen activator in patients with evolving myocardial infarction", & N. ENGL. J. MED., 310(10), 609-613, 1984

BIOLOGICAL ABSTRACTS, Band 72, Nr.7, 1981, Seite 4923, Ref.Nr. 47354, Philadelphia, US; C. MATTSSON et al.: "Dissolution of thrombi by tissue plasminogen activator, urokinase and streptokinase in an artificial circulating system", & THROMB. RES., 21(6), 535-546, 1981

BIOLOGICAL ABSTRACTS, Band 71, Nr. 12, 1981, Seite 8662, Ref.Nr. 82660, Philadelphia, US; J. LASIERRA et al.: "Plasmin inhibitors in different fibrinolytic treatment patterns", & HAEMOSTASIS, 10(1), 51-62, 1981

BIOLOGICAL ABSTRACTS, Band 76, Nr. 12, 1983, Seite 9875, Ref.Nr. 89914, Philadelphia, US; H. HASEGAWA et al.: "Role of dextran sulfate in urokinase therapy and evaluation of the effects by estimation of plasmin inhibitor, fibrinogenolytic degradation products and fibrinolytic degradation products", && JPN. HEART J., 23(3), 339-348, 1982

BIOLOGICAL ABSTRACTS, Band 73, Nr. 6, 1982, Seite 4253, Ref.Nr. 41278, Philadelphia, US; O. MATSUO et al.: "Comparison of the relative fibrinogenolytic, fibrinolytic an thrombolytic properties of tissue plasminogen activator and urokinase in vitro", && THROMB. HAEMOSTASIS, 45(3), 225-229, 1981

BIOLOGICAL ABSTRACTS, Band 70, Nr. 8, 1980, Seite 5557, Ref.Nr. 52869, Philadelphia, US; A. TAKADA et al.: "Interaction of plasmin with tranexamic acid and alpha-2-plasmin inhibitor in the plasma and clot", & THROMB. HAEMOSTASIS, 43(1), 20-23, 1980

Dorland's Illustrated Medical Dictionary, 26th ed.(1981), Seiten 52, 93, 94; Haematology, 32 ed. (1983), Seiten 1268-1269, Arzneimittelwirkungen, 5 Auflage (1986), Seite 407; Barrett and Salvesen, Proteinase Inhibitors (1986), Seiten 457-476.

**Beschreibung**

Die Erfindung betrifft ein Mittel enthaltend einen Plasmin-Inhibitor (PI) und einen Plasminogen-Aktivator (PA) für die Behandlung von Thrombosen.

Streptokinase wird für die fibrinolytische Behandlung von Thrombosen seit mehr als 20 Jahren erfolgreich eingesetzt. Nebenwirkungen limitieren jedoch die Streptokinase-Therapie auf begrenzte Indikationsgebiete. Zu diesen Nebenwirkungen gehört auch der Verbrauch von Gerinnungsfaktoren und Komponenten des fibrinolytischen Systems wie Fibrinogen, Faktor V, Plasminogen, $alpha_2$-Antiplasmin und $alpha_2$-Makroglobulin. Dieser Verbrauch wie auch die Bildung von Fibrinogen-Spaltprodukten verursachen Blutungs- oder Rethrombosierungsrisiken. Diese Störungen sind auf die Bildung von freiem zirkulierenden Plasmin zurückzuführen. Freigesetztes Plasmin wird zuerst durch Plasma-Inhibitoren, insbesondere $alpha_2$-Antiplasmin und $alpha_2$-Makroglobulin, gehemmt. Nach Erschöpfung des Inhibitor-Potentials ist das Plasmin in der Lage, zahlreiche Proteine abzubauen. Plasmin führt durch Histamin-und Kininliberierung zur Erhöhung der Gefäßpermeabilität und Blutdrucksenkung sowie zum Fibrinogenabbau und zu einem Blutungsrisiko. Die für die Streptokinase beschriebenen Nebenreaktionen können auch von Urokinase sowie in begrenztem Maße von Gewebsplasminogen-Aktivator (tPA) verursacht werden. Es wurde bereits versucht, diese Nebenreaktionen mit acylierten Streptokinase-Plasminogen-Komplexen zu vermeiden. Die schnelle Deacylierung dieser Komplexe in vivo vereitelte den Erfolg und führte zu den gleichen Nebenwirkungen wie oben für Streptokinase und Urokinase erwähnt.

Alpha$_2$-Antiplasmin und $alpha_2$-Makroglobulin sind natürlich vorkommende, Plasmin-Inhibitoren. Der Antiplasmin-Inhibitor besitzt eine hohe Affinität zu Fibrin und kann sich somit an der Thrombusoberfläche anlagern. Weiterhin wird er durch aktivierten F XIII kovalent an Fibrin gebunden. Diese Eigenschaften schützen das Gerinnsel vor Plasminabbau. Die Zugabe von $alpha_2$-Antiplasmin während der Infusion von Streptokinase in Katzen führt zur Normalisierung der hämostatischen Parameter wie PTT und TZ (Thromb. Res. 30, 205-212, 1983). Da Plasmin als wesentliche Komponente bei der Fibrinolyse angesehen wird, würde man jedoch erwarten, daß ein von außen zugeführter Plasmin-Inhibitor aufgrund der oben beschriebenen Eigenschaften die Lyse des Thrombus verzögert.

Aus Biol. Abstr. 64: 60894 ist ein Mittel zur therapeutischen Anwendung enthaltend menschliches Glu-Plasminogen, Urokinase und Leupeptin, $alpha_1$-Antitrypsin oder Antithrombin III (dazu Heparin) in einem 25 %igen Glycerol Puffer bekannt.

Aus Biol. Abstr. 72: 47354 geht hervor, daß sich t-PA, Urokinase und Streptokinase zur Lösung eines Gerinnsels eignen.

Biol. Abstr. 71: 82660 ist zu entnehmen, daß sich Plasmin-Inhibitoren, Urokinase und Streptokinase zur Behandlung von Thrombosen eignen.

Gegenstand der Erfindung ist somit ein Mittel zur Lyse-Therapie enthaltend einen Plasmin-Inhibitor Vorzugsweise Antiplasmin oder $alpha_2$-Makroglobulin und einen Plasminogen-Aktivator.

Gegenstand der Erfindung ist insbesondere ein Arzneimittel enthaltend beispielsweise 300 bis 30.000 Einheiten $alpha_2$-Antiplasmin oder 10 bis 30.000 mg $alpha_2$-Makroglobulin pro Dosis (für 75 kg Körpergewicht) und einen Plasminogen-Aktivator (Eine Einheit $alpha_2$-Antiplasmin ist als die Antiplasmin-Aktivität eines Milliliters Standard-Human-Plasma definiert.).

Falls es zweckmäßig ist, kann das Mittel auch mehr als einen Plasminogen-Aktivator sowie auch mehr als einen Plasmin-Inhibitor enthalten.

Unter dem Begriff Plasminogen-Aktivator soll besonders Streptokinase, acylierte Streptokinase, Streptokinase-Plasminogen-Komplex, Pro-Urokinase, Urokinase oder tPA verstanden werden.

Ein solches Mittel wird vorzugsweise in Form eines Lyophilisats hergestellt. Da die Kombination von einem PI und einem Plasminogen-Aktivator in dem beanspruchten Mittel den beschriebenen Zweck im Organismus erfüllen soll, wozu die gleichzeitige Verabreichung der beiden Komponenten keine unabdingbare Voraussetzung zu sein braucht, kann das Arzneimittel auch aus getrennten Abfüllungen bestehen. Sie müssen lediglich dem Organismus in einem solchen zeitlichen Zusammenhang zugeführt werden, daß der gewünschte Zweck erreicht wird.

Ein solches Mittel kann Stabilisatoren wie Albumin, Gelatine, Salze, Zucker oder Aminosäuren enthalten.

Die Verwendung von einem PI zusammen mit einem Plasminogen-Aktivator, beispielsweise als Bolus oder Infusion, zur Lyse-Therapie dient dazu, die hämostatischen Parameter auf dem Normalwert zu halten und somit eine Blutungsgefahr zu vermeiden.

Gegenstand der Erfindung ist auch ein Verfahren zur Herstellung eines Arzneimittels zur Lyse-Therapie, dadurch gekennzeichnet, daß eine einen Plasmin-Inhibitor und einen Plasminogen-Aktivator enthaltende Lösung hergestellt wird, gegebenenfalls als Stabilisatoren Albumin, Gelatine, Salze, Zucker und/oder Aminosäuren zugesetzt werden und gegebenenfalls zur Trockene gebracht wird.

Gegebenenfalls können die Lösungen der Komponenten auch getrennt hergestellt, mit den Stabilisatoren versetzt und gegebenenfalls zur Trockene gebracht werden.

Gegenstand der Erfindung ist schließlich auch die Verwendung von einem Plasmin-Inhibitor und einem Plasminogen-Aktivator in einem Verfahren zur Herstellung eines Arzneimittels zur Lyse-Therapie.

Die Vorteilhaftigkeit einer Verwendung von alpha$_2$-Antiplasmin zusammen mit einem Plasminogen-Aktivator zur Lyse-Therapie geht aus der folgenden Tabelle hervor.

Dazu wurde ein 1 ml Standard-Fibrin-Gerinnsel, enthaltend 4 CTA/ml Human-Plasminogen mit 4 ml Human-Plasma 30 Minuten inkubiert. Danach wurden 300 Einheiten Streptokinase/ ml Plasma und 0 oder 2 Einheiten/ml alpha$_2$- Antiplasmin zugesetzt. Nach 120 Minuten wurden Proben entnommen und PTT, TZ, Fibrinogen, Plasmin sowie das Ausmaß der Auflösung der Gerinnsel bestimmt.

Tabelle 1

| SK E/ml | API E/ml | TZ (sec) | PTT (sec) | Fibrinogen (mg/ml) | Gerinnselauflösung (%) |
|---------|----------|----------|-----------|--------------------|------------------------|
| 0 | 0 | 23,6 | 49,0 | 2,80 | 0 |
| 300 | 0 | 142,5 | 123,1 | 0,10 | 100 |
| 300 | 2 | 31,4 | 48,5 | 1,80 | 97,6 |

Abkürzungen: SK = Streptokinase, API = alpha$_2$-Antiplasmin, TZ = Thrombinzeit, PTT = partielle Thromboplastin-Zeit, CTA = Plasmin-Aktivität in Einheiten des "Committee on Thrombolytic Agents".

Wie aus der Tabelle ersichtlich ist, normalisiert der Zusatz von alpha$_2$-Antiplasmin die Gerinnungsparameter, während die Gerinnselauflösung nicht beeinflußt wird.

Wenn alpha$_2$-Makroglobulin anstelle von Antiplasmin gebraucht wurde, wurden vergleichbare Ergebnisse gefunden.

Beispiel 1

Herstellung eines Arzneimittels enthaltend einen Plasminogen-Aktivator und alpha$_2$-Antiplasmin

5 ml einer Lösung enthaltend Streptokinase (10.000 E/ml) und 1 mg/ml Human-Albumin wurden mit 5 ml einer Lösung enthaltend 200 E/ml Antiplasmin gemischt, 16 Std. bei 4°C gegen 2 l einer gepufferten Lösung (0,05 M Phosphat, 0,1 M NaCl, pH 7,2) dialysiert, anschließend sterilfiltriert und gefriergetrocknet.

Beispiel 2

Herstellung eines Arzneimittels enthaltend einen Plasminogen-Aktivator und alpha$_2$-Makroglobulin

5 ml einer Lösung enthaltend Streptokinase (10.000 E/ml) und 1 mg/ml Human-Albumin wurden mit 5 ml einer Lösung enthaltend 20 mg/ml Makroglobulin gemischt, 16 Stunden bei 4°C gegen 2 l einer gepufferten Lösung (0,05 M Phosphat, 0,1 M NaCl, pH 7,2) dialysiert, anschließend sterilfiltriert und gefriergetrocknet.

**Patentansprüche**

1. Mittel enthaltend einen Plasmin-Inhibitor und einen Plasminogen-Aktivator und gegebenenfalls Hilfsstoffe für die Behandlung von Thrombosen.

2. Mittel nach Anspruch 1, dadurch gekennzeichnet, daß der Plasmin-Inhibitor Antiplasmin ist.

3. Mittel nach Anspruch 1, dadurch gekennzeichnet, daß der Plasmin-Inhibitor alpha$_2$-Makroglobulin ist.

4. Mittel nach Anspruch 1 enthaltend 300 bis 30.000 Einheiten alpha$_2$-Antiplasmin pro Dosis (für 75 kg Körpergewicht) und einen Plasminogen-Aktivator.

**5.** Mittel nach Anspruch 1 enthaltend 10 bis 20.000 mg alpha$_2$-Makroglobulin pro Dosis (für 75 kg Körpergewicht) und einen Plasminogen-Aktivator.

**6.** Mittel nach Anspruch 1 enthaltend als Plasminogen-Aktivator Streptokinase, oder eines ihrer biologisch aktiven Analoga.

**7.** Mittel nach Anspruch 1 enthaltend als Plasminogen-Aktivator Pro-Urokinase oder eines ihrer biologisch aktiven Analoga.

**8.** Mittel nach Anspruch 1 enthaltend als Plasminogen-Aktivator Urokinase oder eines ihrer biologisch aktiven Analoga.

**9.** Mittel nach Anspruch 1 enthaltend Gewebeplasminogen-Aktivator (tPA) oder eines seiner Analoga.

**10.** Verfahren zur Herstellung eines Mittels nach Anspruch 1, dadurch gekennzeichnet, daß eine einen Plasmin-Inhibitor und einen Plasminogen-Aktivator enthaltende Lösung hergestellt wird, gegebenenfalls Albumin, Gelatine, Salze, Zucker und/oder Aminosäuren zugesetzt werden und gegebenenfalls zur Trockene gebracht wird.

**11.** Verfahren zur Herstellung eines Mittels zur Lyse-Therapie, dadurch gekennzeichnet, daß eine einen Plasmin-Inhibitor und eine einen Plasminogen-Aktivator enthaltende Lösung hergestellt werden, denen gegebenenfalls Albumin, Gelatine, Salze, Zucker und/oder Aminosäuren zugesetzt werden und diese zur Trockene gebracht werden.

**12.** Verwendung von einem Plasmin-Inhibitor und einem Plasminogen-Aktivator in einem Verfahren zur Herstellung eines Arzneimittels zur Lyse-Therapie.

**Claims**

**1.** An agent containing a plasmin inhibitor and a plasminogen activator and, where appropriate, auxiliaries for the treatment of thromboses.

**2.** An agent as claimed in claim 1, wherein the plasmin inhibitor is antiplasmin.

**3.** An agent as claimed in claim 1, wherein the plasmin inhibitor is alpha$_2$-macroglobulin.

**4.** An agent as claimed in claim 1, containing 300 to 30,000 units of alpha$_2$-antiplasmin per dose (for 75 kg body weight) and a plasminogen activator.

**5.** An agent as claimed in claim 1, containing 10 to 20,000 mg of alpha$_2$-macroglobulin per dose (for 75 kg body weight) and a plasminogen activator.

**6.** An agent as claimed in claim 1, containing streptokinase, or one of its biologically active analogs, as plasminogen activator.

**7.** An agent as claimed in claim 1, containing prourokinase, or one of its biologically active analogs, as plasminogen activator.

**8.** An agent as claimed in claim 1, containing urokinase, or one of its biologically active analogs, as plasminogen activator.

**9.** An agent as claimed in claim 1, containing tissue plasminogen activator (tPA) or one of its analogs.

**10.** A process for the preparation of an agent as claimed in claim 1, which comprises preparation of a solution containing a plasmin inhibitor and a plasminogen activator, where appropriate addition of albumin, gelatine, salts, sugars and/or amino acids and, where appropriate, drying being carried out.

**11.** A process for the preparation of an agent for lysis therapy, which comprises preparation of a solution containing a plasmin inhibitor and of a solution containing a plasminogen activator, to each of which, where appropriate, albumin, gelatine, salts, sugars and/or amino acids are added, and the latter are dried.

12. The use of a plasmin inhibitor and a plasminogen activator in a process for the preparation of a medicament for lysis therapy.

**Revendications**

1. Agent contenant un inhibiteur de la plasmine et un activateur du plasminogène et éventuellement des adjuvants, pour le traitement de thromboses.

2. Agent selon la revendication 1, caractérisé en ce que l'inhibiteur de la plasmine est l'antiplasmine.

3. Agent selon la revendication 1, caractérise en ce que l'inhibiteur de la plasmine est l'$\alpha_2$-macroglobuline.

4. Agent selon la revendication 1, contenant de 300 à 30 000 unités d'$\alpha_2$-antiplasmine par dose (pour un poids corporel de 75 kg) et un activateur du plasminogène.

5. Agent selon la revendication 1, contenant de 10 à 20 000 mg d'$\alpha_2$-macroglobuline par dose (pour un poids corporel de 60 kg) et un activateur du plasminogène.

6. Agent selon la revendication 1, contenant, en tant qu'activateur du plasminogène, de la streptokinase ou l'un de ses analogues biologiquement actifs.

7. Agent selon la revendication 1, contenant, en tant qu'activateur du plasminogène, de la pro-urokinase ou l'un de ses analogues biologiquement actifs.

8. Agent selon la revendication 1, contenant, en tant qu'activateur du plasminogène, de l'urokinase ou l'un de ses analogues biologiquement actifs.

9. Agent selon la revendication 1, contenant de l'activateur tissulaire de plasminogène (tPA) ou l'un de ses analogues.

10. Procédé pour la préparation d'un agent selon la revendication 1, caractérisé en ce que l'on prépare une solution contenant un inhibiteur de la plasmine et un activateur du plasminogène, on y ajoute éventuellement de l'albumine, de la gélatine, des sels, des sucres et/ou des aminoacides, et éventuellement on la porte à siccité.

11. Procédé pour la préparation d'un agent pour la thérapie de lyse, caractérisé en ce que l'on prépare une solution contenant un inhibiteur de la plasmine et une solution contenant un activateur du plasminogène, auxquelles on ajoute éventuellement de l'albumine, de la gélatine, des sels, des sucres et/ou des aminoacides, et on les porte à siccité.

12. Utilisation d'un inhibiteur de la plasmine et d'un activateur du plasminogène, dans un procédé pour la fabrication d'un médicament pour la thérapie de lyse du thrombus.